(19) Europäisches Patentamt
European Patent Office
Office européen des brevets

(11) Publication number: 0 060 121 B1

(12) **EUROPEAN PATENT SPECIFICATION**

(45) Date of publication of patent specification: **05.12.84**

(21) Application number: **82301167.1**

(22) Date of filing: **08.03.82**

(51) Int. Cl.³: **C 07 C 103/52,** A 01 N 65/00, C 12 P 1/00, C 12 P 21/04 // (C12P1/00, C12R1/89),(C12P21/04, C12R1/89)

(54) **Cyclic peptide and its use as a fungicide.**

(30) Priority: **09.03.81 US 241812**

(43) Date of publication of application: **15.09.82 Bulletin 82/37**

(45) Publication of the grant of the patent: **05.12.84 Bulletin 84/49**

(84) Designated Contracting States: **BE CH DE FR GB IT LI LU NL SE**

(56) References cited:
**TETRAHEDRON LETTERS, no.22, May 1979, Pergamon Press, Oxford (GB) C.J.SIMMONS et al.:"Pukeleimide C, a novel pyrrolic compound from the marine cyanophyte lyngbya majuscula", pages 2003-2006.**
**TETRAHEDRON, vol.36, no.8, 1980, Pergamon Press, Oxford (GB) J.H. CARDELLINA et al.:"Malyngic acid, a new fatty acid from lyngbya majuscula", pages 993-995.**

**The file contains technical information submitted after the application was filed and not included in this specification**

(73) Proprietor: **UNIVERSITY OF HAWAII**
**Honolulu Hawaii (US)**

(72) Inventor: **Moore, Richard Elliott**
**4494 Pahoa Avenue**
**Honolulu Hawaii 96811 (US)**
Inventor: **Mynderse, John Stuart**
**4025 Rommel Drive**
**Indianapolis Indiana 46208 (US)**

(74) Representative: **Hudson, Christopher Mark**
**Erl Wood Manor**
**Windlesham Surrey GU20 6PH (GB)**

(56) References cited:
**THE JOURNAL OF ORGANIC CHEMISTRY, vol.42, no.17, 1977, American chemical society, F.J. MARNER et al.:"Majusculamides A and B, two epimeric lipodipeptides from lyngbya majuscula gomont", pages 2815-2819.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 060 121 B1

## Description

This invention relates to a new cyclic peptide which is designated majusculamide C. We have found that majusculamide C is contained in marine organisms, in particular in an algal association, the primary constituent of which is the blue-green alga *Lyngbya majuscula.* This algal association can be collected from Pinnacles in Enewetak lagoon at Enewetak atoll, which is located in the Marshall Islands.

Accordingly, this invention concerns the isolation of majusculamide C from natural sources, especially marine organisms, particularly a deep-water strain of blue-green alga of the species *Lyngbya majuscula* (see T. V. Desikachary "The Biology of Blue-Green Algae" edited by N. G. Carr and B. A. Whitton, University of California Press, Berkeley, California 1973 (page 473). Majusculamide C in substantially pure form, i.e., free from naturally occurring by-products, is a further feature of the present invention. In other aspects, this invention concerns the use of majusculamide C to inhibit fungal plant pathogens and to fungicidal compositions containing majusculamide C.

Fungal plant pathogens cause great economic losses each year. New agents effective against these phytopathogens are needed. Presently used fungicides differ in their effectiveness against specific fungi. In addition, resistant fungal strains frequently develop, creating a continual need for new, effective agents.

This invention provides a new cyclic peptide compound which is designated majusculamide C. Majusculamide C is useful against a number of fungal plant pathogens such as, for example, tomato late blight and grape downy mildew.

Majusculamide C has thus far been found in an algal association, the primary constituent of which is a deep-water seaweed of the family Oscillatoriaceae, the blue-green alga *Lyngbya majuscula.* The term "deep water" used herein refers to depths of from about 25 to about 100 feet. This term is used to distinguish the algal association of this invention from *Lyngbya majuscula* found in the shallow waters, i.e. depths of less than about 20 feet, of Enewetak lagoon. The shallow-water *Lyngbya* found there do not contain majusculamide C.

The alga of this invention is found in the lagoon at Enewetak atoll in the Marshall Islands. In addition to majusculamide C, the alga produces a toxin, debromoaplysiatoxin, which causes a severe contact dermatitis called "swimmers' itch" [see Mynderse et al., *Science 196*, 538—540 (1977)]. This toxin is separated from majusculamide C by chromatography.

Majusculamides A and B are major lipophilic constituents of several shallow-water varieties of *Lyngbya majuscula* found in Hawaii and Enewetak [see Marner et al., *J. Org. Chem. 42,* 2815—2819 (1977)]. Majusculamide B is N-[(S)-2-methyl-3-oxodecanoyl]-*D*-N,O-dimethyltyrosyl-*L*-N-methylvalinamide. Majusculamide A is the (2R)-2-methyl-3-oxodecanoyl epimer of majusculamide A. Majusculamides A and B were not found in the deep-water *L. majuscula* which contained majusculamide C.

Majusculamide C can be isolated from the natural source, e.g., from the blue-green alga *Lyngbya majuscula,* using techniques which will be apparent to those skilled in the art. For example, a preferred method of isolating majusculamide C is to extract the freeze-dried alga exhaustively with hexane, acetone, and methanol. The organic solvents are then combined and evaporated to give an extract. The extract is partitioned between hexane and 90% aqueous methanol. The methanolic portion is then partitioned between dichloromethane and 80% aqueous methanol. The dichloromethane portion is chromatographed over Sephadex (LH-20) (Sephadex is a Registered Trade Mark) to give majusculamide C which can be further purified by high performance liquid chromatography (HPLC).

Majusculamide C is a white amorphous solid which has a molecular weight of approximately 984, based on field-desorption mass spectral data.

Majusculamide C is soluble in solvents such as methanol, dichloromethane, and acetone but is not soluble in water. The specific optical rotation, $[\alpha]_D$, of majusculamide C is −96° (*c* 2.5, $CH_2Cl_2$).

The infrared (IR) spectrum of majusculamide C in KBr disc is shown in the accompanying drawing. The IR spectrum has the following absorption maxima:

| Wavenumber ($cm^{-1}$) | Percent transmittance |
|---|---|
| 3336.13 | 46.73 |
| 2967.70 | 38.329 |
| 2937.80 | 46.061 |
| 2878.97 | 53.83 |
| 2837.49 | 68.28 |
| 1741.85 | 40.857 |
| 1680.12 | 15.247 |
| 1643.47 | 9.872 |
| 1585.60 | 56.29 |
| 1514.23 | 16.761 |
| 1465.04 | 34.352 |
| 1411.03 | 43.91 |
| 1385.95 | 47.18 |
| 1373.42 | 49.82 |
| 1357.98 | 52.62 |
| 1301.08 | 44.04 |
| 1289.51 | 43.16 |
| 1249.00 | 36.099 |
| 1180.52 | 43.466 |
| 1142.91 | 48.76 |
| 1127.47 | 47.32 |
| 1109.15 | 50.70 |
| 1086.93 | 51.31 |
| 1033.92 | 49.04 |
| 1000.16 | 57.66 |
| 946.15 | 64.56 |
| 908.54 | 70.48 |
| 857.42 | 69.30 |
| 841.99 | 64.20 |
| 827.52 | 64.63 |
| 812.09 | 66.6 |
| 783.16 | 67.19 |

The ultraviolet absorption spectrum of majusculamide C in ethanol shows absorption maxima at 278 nm (ε 1420) and 230 nm (ε 5900).

$^1$H and $^{13}$C nuclear magnetic resonance (NMR) spectral and mass spectral analyses indicate that the molecular composition of majusculamide C is $C_{50}H_{80}N_8O_{12}$. Nuclear magnetic resonance analysis also shows that two glycyl units, one alanyl unit, one N-methylvalyl unit, and one N,O-dimethyltyrosyl unit are present. Acid hydrolysis (1.5 N HCl, 25% ethanol-water, reflux 24 hours) of majusculamide C leads to *L*-alanine, glycine, *L*-N-methylvaline, *L*-N,O-dimethyltyrosine, *L*-N-methylisoleucine, 2-hydroxy-3-methylpentanoylglycine, 2-amino-3-oxo-4-methylpentane (isolated as the hydrochloride), and 3-amino-2-methylpentanoic acid. Thus, the following eight sub-units (*1a* through *1h*) account for all the atoms in the molecular formula of majusculamide C:

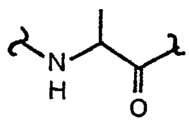

*1a*

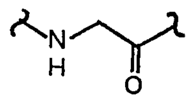

*1b*

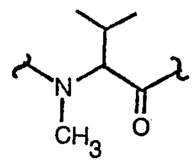

*1c*

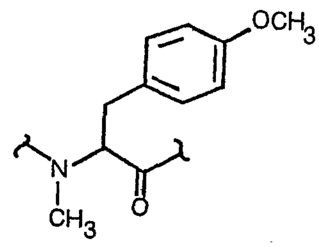

*1d*

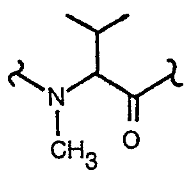

*1e*

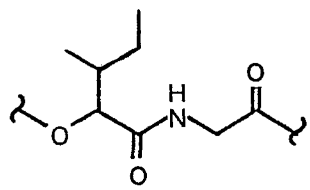

*1f*

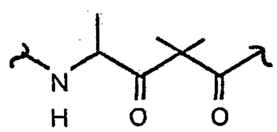

*1g*

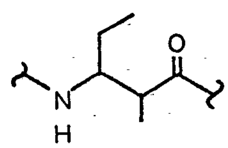

*1h*

Table 1 gives the $^1$H NMR data for majusculamide C at 360 MHz.

TABLE 1
$^1$H NMR Signals of majusculamide C

| Chemical shift* | | Multiplicity** |
|---|---|---|
| 7.767 | | d, J=8.1 Hz |
| 7.573 | | d, J=7.4 Hz |
| 7.355 | | t, J=5.4 Hz |
| 7.309 | | d, J=6.7 Hz |
| 7.129 | 2H, | d, J=8.5 Hz |
| 7.068 | | d, J=10.2 Hz |
| 6.834 | 2H | d, J=8.5 Hz |
| 5.186 | | d, J=3.2 Hz |
| 5.132 | | t, J=7.3 Hz |
| 4.893 | | p, J=6.8 Hz |
| 4.888 | | d, J=11.1 Hz |
| 4.777 | | d, J=10.7 Hz |
| 4.606 | | dd, J=8.3, 17.7 Hz |
| 4.509 | | tdd, J=10.2, 5.7, 2.7 Hz |

TABLE 1 (contd.)
$^1$H NMR Signals of majusculamide C

| Chemical shift* | | Multiplicity** |
|---|---|---|
| 4.437 | | dd, J=15.9, 5.4 Hz |
| 4.401 | | dd, J=2.1, 6.7 Hz |
| 3.738 | 3H, | s, |
| 3.541 | | dd, J=15.9, 5.0 Hz |
| 3.445 | | dd, J=17.6, 1.0 Hz |
| 3.237 | | dd, J=14.2, 7.0 Hz |
| 3.201 | 3H, | s |
| 2.944 | 3H, | s |
| 2.938 | 3H, | s |
| 2.792 | | dd, J=8.1, 14.0 Hz |
| 2.735 | | dq, J=2.6, 7.0 Hz |
| 2.250 | | ds, J=10.7, 6.7 Hz |
| 2.206 | 2H, | m |
| 1.508 | 3H, | s |
| 1.2—1.6 | 6H, | m |
| 1.462 | 3H, | s |
| 1.136 | 3H, | d, J=6.8 Hz |
| 1.082 | 3H, | d, J=7.0 Hz |
| 1.042 | 3H, | d, J=6.6 Hz |
| 1.024 | 3H, | d, J=6.2 Hz |
| 0.916 | 3H, | d, J=7.4 Hz |
| 0.873 | 3H, | d, J=7.5 Hz |
| 0.862 | 6H, | t, J=7.7 Hz |
| 0.738 | 3H, | d, J=6.6 Hz |
| 0.393 | 3H, | d, J=6.5 Hz |

*Chemical shifts are the values in chloroform-*d* in ppm. A signal represents one proton unless otherwise described.

**s=singlet, d=doublet, dd=doublet of doublets, tdd=triplet of doublets of doublets, dq=doublet of quartets, ds=doublet of septets, p=pentet.

The $^{13}$C NMR data for majusculamide C are listed in Table 2.

TABLE 2
$^{13}C$ NMR Signals of majusculamide C in deuteriochloroform*

| | | | | |
|---|---|---|---|---|
| 209.97(s) | 158.58(s) | 55.06(s) | 32.63(d) | 18.95(q) |
| 172.64(s) | 130.28(d) | 54.72(s) | 30.17(s) | 18.32(q) |
| 172.46(s) | 130.28(d) | 51.45(d) | 29.08(s) | 18.27(q) |
| 171.90(s) | 128.55(s) | 51.07(d) | 29.04(s) | 15.31(q) |
| 170.91(s) | 114.15(s) | 48.15(d) | 26.86(d) | 15.30(q) |
| 170.12(s) | 114.15(s) | 42.36(d) | 25.85(t) | 15.30(q) |
| 169.99(s) | 78.20(d) | 40.62(t) | 24.80(t) | 11.48(q) |
| 169.86(s) | 60.98(d) | 40.45(t) | 23.62(t) | 10.80(q) |
| 169.21(s) | 60.86(d) | 37.20(d) | 21.98(q) | 9.88(q) |
| 167.85(s) | 57.98(d) | 34.46(t) | 21.30(q) | 9.60(q) |

*Chemical shifts are expressed in ppm.
The multiplicity is in parentheses.

Table 3 lists conclusions which can be drawn about the structure of majusculamide C based on the $^1H$ NMR and $^{13}C$ NMR spectra.

TABLE 3
Structural conclusions for majusculamide C from $^1H$ NMR and $^{13}C$ NMR spectra

| Chemical shift | Number and kinds of carbons |
|---|---|
| 209.97 | 1 Ketone carbonyl |
| 167.85, 169.21, 169.86, 169.99, 170.12, 170.91, 171.90, 172.46, 172.64 | 9 Amide/ester carbonyls |
| 128.55, 158.58 | 2 Aromatic tertiary carbons |
| 114.15(2), 130.28(2) | 4 Aromatic methine carbons |
| 55.06 | 1 Methoxy |
| 29.04, 29.08, 30.17 | 3 N-methyls |
| 54.72 | 1 Tertiary carbon |
| 26.86, 32.63, 37.20, 42.36, 48.15, 51.07, 51.45, 57.98, 60.86, 60.98, 78.20 | 11 Methines |
| 23.62, 24.80, 25.85, 34.46, 40.45, 40.62 | 6 Methylenes |
| 9.60, 9.88, 10.80, 11.48, 15.30, 15.30, 15.31, 18.27, 18.32, 18.95, 21.30, 21.98 | 12 Methyls |

Electron-impact mass spectrometry (low resolution) analysis of majusculamide C gives the following fragmentation pattern, *m/z* (rel intensity): 757(5), 756(13), 597(14), 596(38), 566(21), 565(62), 508(2), 452(6), 396(19), 395(80), 381(2), 324(2), 275(9), 248(5), 211(6), 204(28), 164(12), 161(19), 155(12), 121(13), 114(100%).

The observed and calculated fragments of majusculamide C on high resolution electron impact mass spectroscopy are summarized in Table 4.

TABLE 4
High resolution mass spectral data of majusculamide C

| | Calculated | Observed |
|---|---|---|
| $C_{12}H_{19}O_3$ | 211.13342 | 211.1327 |
| $C_{17}H_{23}O_5N$ | 324.18108 | 324.1786 |
| $C_{21}H_{35}O_5N_2$ | 395.25458 | 395.2529 |
| $C_{23}H_{38}O_6N_3$ | 452.27606 | 452.2730 |
| $C_{29}H_{49}O_7N_4$ | 565.36013 | 565.3570 |
| $C_{40}H_{62}O_9N_5$ | 756.45476 | 756.4455 |

Sequencing based on the mass spectral data suggests that a glycyl-N-methylisoleucylglycyl-N-methylvalyl-N,O-dimethyltyrosyl sequence is present in majusculamide C.

Majusculamide C has a retention time of approximately 14.7 minutes on high performance liquid chromatography (HPLC), using a DuPont Zorbax C-8 (Zorbax is a Registered Trade Mark) 4.6-mm×25-cm column, an acetonitrile:water (15:85) mobile phase, and a flow rate of 2 ml per minute.

Based on the characteristics known thus far, the structure shown in formula *2* has been postulated as the tentative structure of majusculamide C:

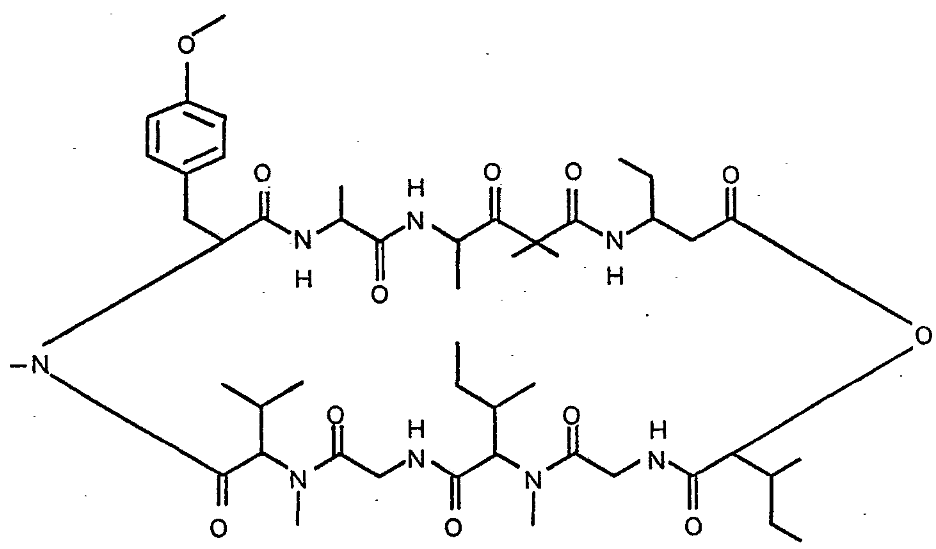

*2*

This invention further concerns a process to produce majusculamide C from a deep-water algal association containing as its primary constituent the blue-green alga *Lyngbya majuscula* which comprises extracting the alga with an organic solvent in which the compound is soluble and thereafter isolating the compound by chromatography.

This invention also concerns a method of inhibiting a plant-pathogenic fungus which comprises applying to the locus of the fungus an effective amount of majusculamide C. The loci of the fungi can be a portion of the plant, such as leaves, stems, flowers or roots, or the soil wherein the fungi may be located. Majusculamide C appears to translocate from roots to shoots; consequently it is possible that application could be made to seeds, roots, etc. to obtain fungicidal effect throughout the plant. Application rates will vary according to a number of factors, such as the location of the plants being protected and the severity of the fungal infection. Thus, for use in a greenhouse, the fungicidal compound is applied as a soil drench using a composition having a concentration in the range of from about 1 to about 200 ppm of active ingredient, preferably from about 5 to about 100 ppm. As is understood by those skilled in the art, application rates used in the field are usually greater than those used in a greenhouse, and range from about 25 to about 1000 ppm.

Majusculamide C has been shown by suitable tests to control a number of fungi, including *Phytophthora infestans,* the causative organism of tomato late blight, *Plasmopora viticola*, the

causative organism of grape downy mildew and *Rhizoctonia solani*, the causative organism of Rhizoctonia damping-off.

In another embodiment, this invention concerns fungicidal formulations comprising as active ingredient an effective amount of majusculamide C associated with one or more agronomically-acceptable carriers or diluents therefore.

The formulations for use in this embodiment desirably contain, in addition to from 0.05 to 95% by weight of the majusculamide C, one or more of a plurality of agronomically-acceptable carriers or diluents including water, polyhydroxy compounds, petroleum distillates, and other dispersion media, surface-active dispersing agents, emulsifiers, and finely-divided inert solids. The concentration of majusculamide C in these compositions will vary depending on whether the composition is intended for direct application to plants or is intended to be subsequently diluted with additional inert carrier such as water to produce the ultimate treating composition.

Treating compositions are most conveniently formulated by preparing an agronomically-acceptable liquid or solid concentrates, which are subsequently diluted to the desired level for use. Emulsifiable liquid concentrates can be prepared by incorporating from about 1 to about 10 percent by weight of the active ingredient and an emulsifiable agent in a suitable water-immiscible organic liquid. Such concentrates may be further diluted with water to form spray mixtures in the form of oil-in-water emulsions. Such spray compositions then comprise active compound, water-immiscible solvent, emulsifying agent, and water. Suitable emulsifying agent can be of the nonionic or ionic types, or blends thereof, and include condensation products of alkylene oxides with phenols and organic acids, polyoxyethylene derivatives of sorbitan esters, complex ether alcohols, ionics of the arylalkyl sulfonate type, and the like. Suitable water-immiscible organic liquids include aromatic hydrocarbons, aliphatic hydrocarbons, cycloaliphatic hydrocarbons, and mixtures thereof such as petroleum distillates.

Solid concentrate mixtures can be prepared by incorporating from about 10 to about 50% by weight of active compound in a finely-divided solid carrier such as bentonite, Fuller's earth, diatomaceous earth, hydrated silica, diatomaceous silica, expanded mica, talc, chalk, and the like. Such concentrates can be formulated, if desired, for direct use as dusting compositions, or can be diluted, if desired, with additional inert solid carriers to produce dusting powders containing around 0.05 to 1% by weight of majusculamide C. Alternatively, the surfactants, that is, dispersing and/or wetting agents, can be incorporated along with majusculamide C in the solid carrier to form wettable powder concentrates ranging from about 10 to about 25% by weight concentration, which subsequently can be dispersed in water or other hydroxylated carrier to form spray compositions. Suitable surfactants include condensed aryl sulfonic acids and sodium salts thereof, sodium lignosulfate, sulfonate-oxide condensate blends, alkylaryl polyether alcohols, sulfonate/nonionic blends, anionic wetting agents, and the like.

Further, majusculamide C can be incorporated in solutions, simple dispersions, aerosol formulations, and other media acceptable for treating vegetation or for applying to the soil.

The antifungal formulation of this embodiment is applied to infected or susceptible plant or soil surfaces in any convenient fashion such as by spraying, dusting, dipping, or drenching. A spray method is considered preferable, especially when large numbers of plants are involved, because such a treatment is faster and more uniform. In spraying it is usually sufficient for the infected or susceptible surfaces to be made thoroughly wet with the liquid dispersion. Good results can be obtained by using spray composition whether they are emulsions or aqueous dispersions of solid concentrates.

Where the fungi to be controlled are in the soil, the antifungal compound can be applied to the soil directly, or it can be diluted with various inert solid or liquid diluents and then applied to the soil. In one method of application the soil surface is sprayed with a liquid dispersion or emulsion of the active ingredient. The application is allowed to remain as a coating on the surface of the soil or, alternatively, is incorporated into the soil by disking, hoeing, or other methods known to those skilled in the art. Another method of application is to apply the active ingredient in the form of a liquid dispersion or emulsion to the soil as a drench. Thus, for the control of soil-inhabiting fungi in the greenhouse, the application rate varies from about 5 to about 200 ppm active ingredient.

Majusculamide C can also be used as a seed soak for seeds prior to planting. A suitable seed soak formulation contains majusculamide C together with excipients such as a mixture of ethanol-acetone, polyoxyethylene sorbitan monolaurate, and the like.

When used as a seed soak, control can be accomplished at an application rate of from about 50 to about 400 ppm of majusculamide C. The seeds are allowed to soak in the formulation for about 4 hours and then are removed and planted.

The activity of majusculamide C against plant pathogenic fungi in standard *in vitro* agar-dilution tests is summarized in Tables 5—10. In these tests 100x solutions of majusculamide C in 95% aqueous ethanol were used. Majusculamide C solution (0.1 ml) and melted agar (9.9 ml) at 50°C were mixed. An inoculum plug (6-mm diameter) was placed in the center of each plate; the radial growth of the colony was measured. Zone of growth was measured from the edge of the plug to the edge of the colony.

TABLE 5

In vitro agar dilution activity of majusculamide C

| Majusculamide C level (ppm) | *Rhizoctonia solani* | | *Sclerotinia homoeocarpa* | | *Pythium aphanidermatum* | |
|---|---|---|---|---|---|---|
| | Zone of growth | Percent inhibition | Zone of growth | Percent inhibition | Zone of growth | Percent inhibition |
| 1 | 19 | 30 | 17 | 26 | 1 | 97 |
| 10 | 10 | 63 | 16 | 30 | 0 | 100 |
| 0[a] | 27[b] | 0 | 23 | 0 | 28 | 0 |

[a]Control

The $ED_{50}$ (effective dose to achieve 50% inhibition) values for majusculamide C against the organisms in Table 5, calculated using the results given there, are summarized in Table 6.

TABLE 6

$ED_{50}$ Values for majusculamide C

| Organism | $ED_{50}$ |
|---|---|
| *Rhizoctonia solani* | 4 ppm |
| *Sclerotinia homoeocarpa* | >10 ppm |
| *Pythium aphanidermatum* | < 1 ppm |

TABLE 7

In vitro agar-dilution activity of majusculamide C and ridomil

| Compound | Level (ppm) | *Pythia aphanidermatum* | | *Aphanomyces euteiches* | | *Phytophthora infestans* | |
|---|---|---|---|---|---|---|---|
| | | Zone of growth | Percent inhibition | Zone of growth | Percent inhibition | Zone of growth | Percent inhibition |
| Majusculamide C | 0.01 | 30 | 6 | 27 | 7 | 10 | 17 |
| Majusculamide C | 0.1 | 12 | 62 | 27 | 7 | 7 | 42 |
| Majusculamide C | 1.0 | 1.5 | 95 | 20.5 | 29 | 6.5 | 46 |
| Majusculamide C | 10.0 | 0 | 100 | 3.5 | 88 | 0 | 100 |
| Ridomil* (tech.) | 0.1 | 21 | 34 | 27 | 7 | 6 | 50 |
| Ridomil* (tech.) | 1.0 | 2 | 94 | 28 | 3 | 2 | 83 |
| Control | 0 | 32 | 0 | 29 | 0 | 12 | 0 |

*metralaxyl (methyl N-(2-methoxyacetyl)-N-(2,6-xylyl)-DL-alaninate).

The $ED_{50}$ values for majusculamide C against the organisms in Table 7, calculated using the results given there, are summarized in Table 8.

TABLE 8
$ED_{50}$ Values for majusculamide C

| Organism | $ED_{50}$ |
|---|---|
| *Pythium aphanidermatum* | 0.07 ppm |
| *Aphanomyces euteiches* | 2.1 ppm |
| *Phytophthora infestans* | 1.07 ppm |

In Table 9 the activity of majusculamide C against two Ridomil-sensitive and two Ridomil-resistant strains is compared.

TABLE 9

In vitro activity of majusculamide C against Ridomil-resistant strains

| Majusculamide C level (ppm) | *Pythium ultimum* | | | | *Phytophthora capsici* | | | |
|---|---|---|---|---|---|---|---|---|
| | Ridomil-sensitive strain | | Ridomil-resistant strain[a] | | Ridomil-sensitive strain | | Ridomil-resistant strain[a] | |
| | Zone of growth | Percent inhibition | Zone of growth | Percent inhibition | Zone of growth | Percent inhibition | Zone of growth | Percent inhibition |
| 0.1 | 18.5 | 53 | 15.5 | 60 | 14.5 | 46 | 14.5 | 48 |
| 1 | 4.5 | 88 | 3.5 | 91 | 7 | 74 | 4.5 | 84 |
| 10 | 0 | 100 | 0[b] | 100 | 0 | 100 | 0.5 | 98 |
| 0[c] | 39 | 0 | 39 | 0 | 27 | 0 | 28 | 0 |

[a]Maintained on V-8 agar with 100 ppm Ridomil.
[b]No growth on plug.
[c]Control.

The $ED_{50}$ values for majusculamide C against the organisms in Table 9, caculated using the results given there, are summarized in Table 10.

TABLE 10

$ED_{50}$ Values for majusculamide C against Ridomil-resistant strains

| Organism | $ED_{50}$ (ppm) |
|---|---|
| *Pythium ultimum* (Ridomil-sensitive) | <0.1 |
| *Pythium ultimum* (Ridomil-resistant) | <0.5 |
| *Phytophthora capsici* (Ridomil-sensitive) | 0.45 |
| *Phytophthora capsici* (Ridomil-resistant) | 0.25 |

Majusculamide C was effective when tested *in vivo* against *Phytophthora infestans*, the causative agent of tomato late blight, and *Plasmopora viticola*, the causative agent of grape downy mildew. In these tests majusculamide C was formulated in a solvent of acetone:ethanol (1:1) plus 0.05% Tween 20 (Tween is a Registered Trade Mark). Tomato leaves (C.V. Rutgers) were placed, top side up, on rubber mats in Petri dishes. Grape leaves (C.V. Niagara) were placed, bottom side up, on filter paper on rubber mats in Petri dishes. Foliar spray solutions of test compounds were placed on the leaves, using two replicates per dosage per leaf type. Leaves were dried, and water (approximately 10 ml) was added to the bottom of the Petri dish. Tomato leaves were inoculated with a zoospore suspension of *P. infestans.* Grape leaves were inoculated with a sporangial suspension of *P. viticola.* Tomato leaves were incubated in a 15°C incubator for 48 hr and then at room temperature for 5 days. Grape leaves were incubated in a lighted chamber at 20°C for 7 days.

Leaves were rated on a 1—5 scale wherein 1=severe disease and 5=no disease. Phytotoxicity was also rated on a 1—5 scale with 0=no injury and 5=severe injury.

Table 11 summarizes the results of these tests.

TABLE 11

In vivo activity of majusculamide C

| Majusculamide C dosage (ppm) | Disease ratings[a] | | | |
|---|---|---|---|---|
| | Tomato late blight | | Grape downy mildew | |
| | Disease control | Phyto-toxicity | Disease control | Phyto-toxicity |
| 400 | 5 | 4[b] | 4+ | 0 |
| 100 | 5 | 3.5[b] | 4− | 0 |
| 25 | 5 | 0 | 3 | 0 |
| 6.25 | 5 | 0 | 2 | 0 |
| 0[c] | 5 | 0 | 1 | 0 |

[a]Average of two replicates.
[b]Evidence of chlorosis.
[c]Control.

In order to illustrate this invention more fully, the following examples are provided.

Example 1

The starting material is a blue-green alga species, *Lyngbya majuscula*, which can be collected in the lagoon on Enewetak atoll. The *L. majuscula* used in this Example was collected from Reefer 8 Pinnacle, Enewetak lagoon, at depths of from about 10.7 to about 18.3 m (about 35 to about 60 feet).

The alga is reddish in color and grows in hair-like strands. The alga is rinsed with sea water to eliminate major contaminating organisms.

Frozen *L. majuscula* (3 kg wet weight) thus collected was homogenized and extracted with a mixture of chloroform and methanol (1:2 by volume). Water was added to the filtrate; the chloroform layer was washed repeatedly with water, then was dried over anhydrous sodium sulfate and evaporated to give a crude extract (22 g). This extract (dissolved in chloroform) was chromatographed over a 4.7-×40-cm Florosil (Registered trade Mark) column ($<74\times10^{-6}$ m operative size ($<200$ U.S. Standard mesh).

The column was developed with hexane (400 ml), hexane:chloroform mixtures (9:1—200 ml; 3:1—300 ml; 1:1—400 ml), chloroform (400 ml) and chloroform:methanol mixtures (9:1—400 ml; 3:1—400 ml; 1:1—200 ml). The following fractions were collected:

| Fraction No. | Volume (ml) |
|---|---|
| 0 | 250 |
| 1 | 300 |
| 2 | 150 |
| 3 | 300 |
| 4 | 250 |
| 5 | 250 |
| 6 | 225 |
| 7 | 250 |
| 8 | 250 |
| 9 | 600 |
| 10 | 250 |

Fractions 5 and 6, collected using hexane:chloroform (1:1) and chloroform, provided, after evaporation, 2.14 g and 1.94 g of substantially pure majusculamide C, respectively.

Subsequent development of the Florosil column, using chloroform:methanol (9:1) gave crude debromoaplysiatoxin which was purified over a silica gel column to give 400 mg of pure debromoaplysiatoxin.

Chromatography of fraction #5 from the Florisil column over a 1.5-cm×1.15-m column of Sephadex LH-20, eluting with chloroform:methanol (1:1) and collecting fractions based on proton magnetic resonance data, gave first fatty compounds and chlorophyll and then gave majusculamide C in an especially pure form (1.69 g).

Example 2

The *L. majuscula* used in this experiment was collected at Enewetak from the South Medren Pinnacle in Enewetak lagoon in depths ranging from 35 to 60 feet. Freeze-dried *L. majuscula* (748 g) was exhaustively extracted with hexane, acetone, and methanol, respectively; the extracts were combined and dried to give 19 g of extract. This crude extract was partitioned between hexane (500 ml) and 90% methanol (100 ml). The resulting methanol layer was diluted with water and then was extracted with dichloromethane (500 ml). The dichloromethane was evaporated to give 4.8 g of brown semisolid which represented 25% of the crude extract, or 0.6% of the dried seaweed.

Majusculamide C was isolated from the dichloromethane extract by gel filtration. A portion of the dichloromethane extract (1.5 to 2 g) was filtered through a 180-×2.5-cm LH-20 Sephadex column, using $CH_2Cl_2$:$CH_3OH$ (1:1) as the solvent system and collecting 50-ml fractions. Crude majusculamide C was eluted in the third fraction.

Majusculamide C was further purified by HPLC with a Whatman Partisil ODS-2 (Partisil is a Registered Trade Mark) reverse phase column. Purification was achieved using 30 mg of crude majusculamide C, a $CH_3CN$:$H_2O$ (7:3) mobile phase, a pressure of 2.11 Pa (21.1 kg/cm$^2$) and a flow rate of 1 ml per minute. Majusculamide C was retained for about 100 to 110 minutes. The eluant yielded a clear amorphous solid upon evaporation.

**Claims**

1. Majusculamide C, which is a white amorphous solid having these characteristics:

a) a molecular weight of approximately 984;
b) an empirical formula of about $C_{50}H_{80}N_8O_{12}$;
c) a specific optical rotation, $[\alpha]_D$, of $-96°$ (*c* 2.5, $CH_2Cl_2$);
d) an infrared spectrum as shown in the accompanying drawing;
e) an ultraviolet absorption spectrum in ethanol with absorption maxima at 278 nm ($\varepsilon$ 1420) and 230 nm ($\varepsilon$ 5900);
f) is soluble in solvents such as methanol, dichloromethane, and acetone, but is insoluble in water;
g) contains structural sub-units having the formula 1*a*—1*h*:

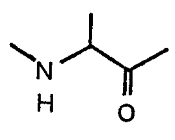
*1a*

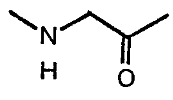
*1b*

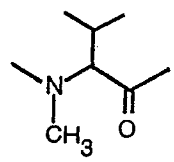
*1c*

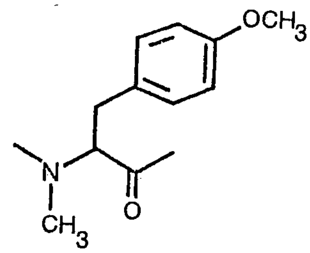
*1d*

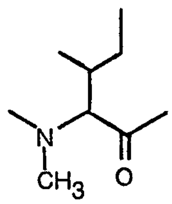
*1e*

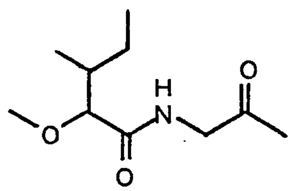
*1f*

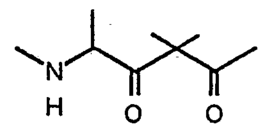
*1g*

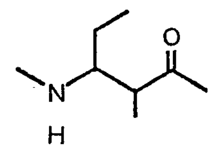
*1h*

h) appears to contain the sub-unit grouping: glycyl-N-methylisoleucylglycyl-N-methylvalyl-N,O-dimethyltyrosyl; and
i) the following tentative structure:

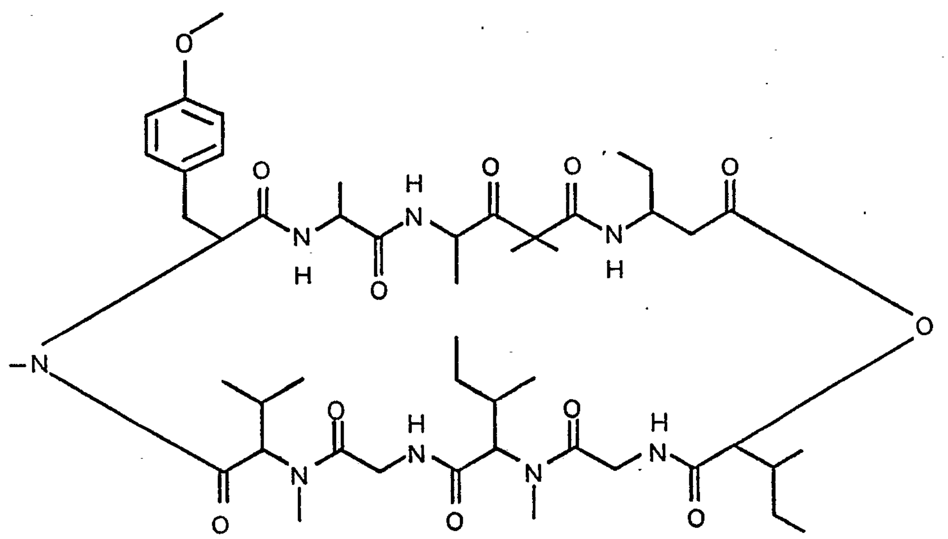
2

2. Majusculamide C as claimed in claim 1 for use as a fungicide.

3. A fungicidal formulation comprising as active ingredient an effective amount of majusculamide C as claimed in claim 1 associated with one or more agronomically-acceptable carriers or diluents therefore.

4. A fungicidal formulation as claimed in claim 3 which is an agronomically-acceptable concentrate.

5. A method of inhibiting a plant-pathogenic fungus which comprises applying to the locus of the fungus an effective amount of the compound of claim 1.

6. A process to produce the compound of claim 1 from a deep-water algal association containing as its primary constituent the blue-green alga *Lyngbya majuscula* which comprises extracting the alga with an organic solvent in which the compound is soluble and thereafter isolating the compound by chromatography.

**Patentansprüche**

1. Majusculamid C, dadurch gekennzeichnet, daß es sich hierbei um einen weißen amorphen Feststoff handelt, der folgende Eigenschaften hat:

a) ein Molekulargewicht von etwa 984,
b) eine empirische Formel von etwa $C_{50}H_{80}N_8O_{12}$,
c) einen spezifischen optischen Drehwert $[\alpha]_D$ von $-96°$ (c=2,5, $CH_2Cl_2$),
d) ein Infrarotspektrum gemäß beigefügter Zeichnung,
e) ein Ultraviolett-Absorptionsspektrum in Ethanol mit Absorptionsmaxima bei 278 nm ($\varepsilon$=1420) und 230 nm ($\varepsilon$=5900),
f) eine Löslichkeit in Lösungsmitteln wie Methanol, Dichlormethan und Aceton, jedoch eine Unlöslichkeit in Wasser,
g) einen Gehalt an Strukturuntereinheiten mit den folgenden Formeln 1a bis 1h:

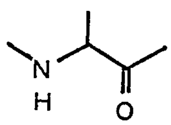
*1a*

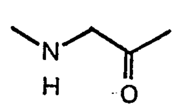
*1b*

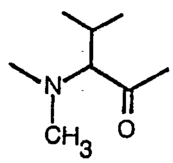
*1c*

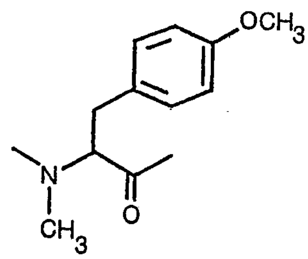
*1d*

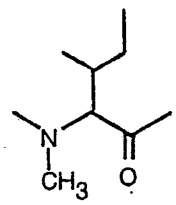
*1e*

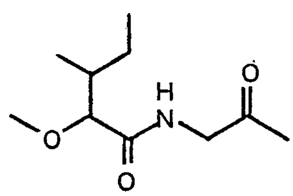
*1f*

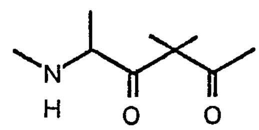
*1g*

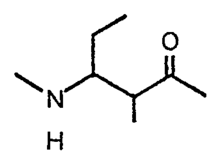
*1h*

h) einen anscheinenden Gehalt an der Untereinheitgruppierung Glycyl-N-methylisoleucylglycyl-N-methylvalyl-N,O-dimethyltyrosyl und

i) die folgende angenommene Struktur:

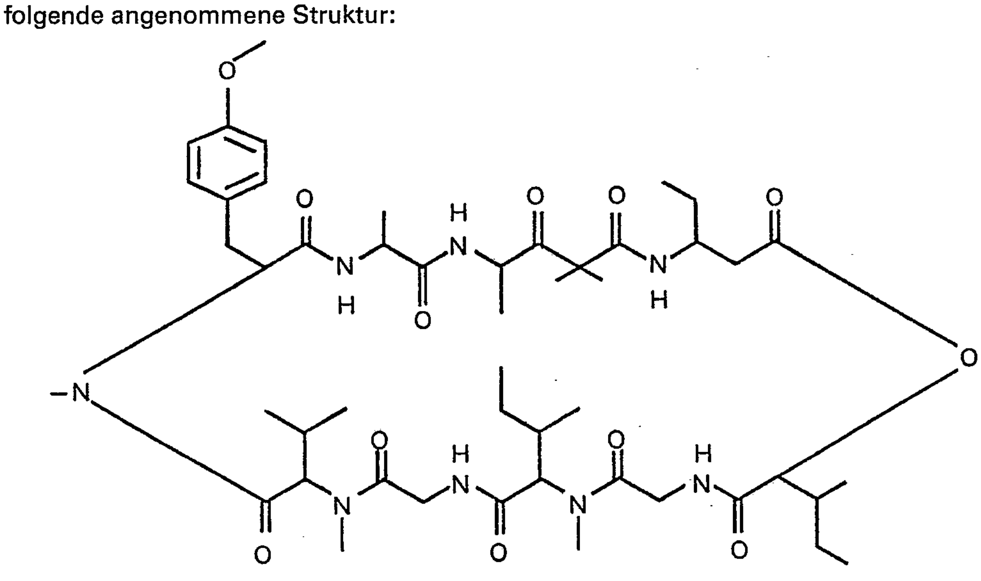

2

2. Verwendung des Majusculamids C gemäß Anspruch 1 als Fungicid.

3. Fungicide Formulierung, dadurch gekennzeichnet, daß sie eine wirksame Menge an Majusculamid C gemäß Anspruch 1 als Wirkstoff in Verbindung mit einem oder mehreren landwirtschaftlich annehmbaren Trägern oder Verdünnungsmitteln hierfür enthält.

4. Fungicide Formulierung gemäß Anspruch 3, dadurch gekennzeichnet, daß sie in Form eines landwirtschaftlich annehmbaren Konzentrats vorliegt.

5. Verfahren zur Hemmung eines krankheitserregenden Pilzes bei Planzen, dadurch gekennzeichnet, daß man die Stelle des jeweiligen Pilzes mit einer wirksamen Menge der Verbindung von Anspruch 1 behandelt.

6. Verfahren zur Herstellung der Verbindung von Anspruch 1 aus einem Tiefwasseralgenassoziat, das als vorweigenden Bestandteil die blaugrüne Alge Lyngbya majuscula enthält, dadurch gekennzeichnet, daß man die Alge mit einem organischen Lösungsmittel, in welchem die Verbindung löslich ist, extrahiert und die Verbindung dann durch Chromatographie isoliert.

## Revendications

1. Majusculamide C qui est un solide amorphe blanc présentant les caractéristiques suivantes:

a) un poids moléculaire d'environ 984;
b) une formule empirique d'environ $C_{50}H_{80}N_8O_{12}$;
c) une rotation optique spécifique $[\alpha]_D$ de $-96°$ (*c* 2,5, $CH_2Cl_2$);
d) un spectre d'absorption des rayons infrarouges du type indiqué dans le dessin annexé;
e) un spectre d'absorption des rayons ultraviolets dans l'éthanol avec des maxima d'absorption à 278 nm ($\varepsilon$ 1.420) et 230 nm ($\varepsilon$ 5.900);
f) il est soluble dans des solvants tels que le méthanol, le dichlorométhane et l'acétone, mais il est insoluble dans l'eau;
g) il contient les sous-unités structurales répondant aux formules *1a—1h*:

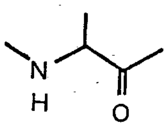

*1a*

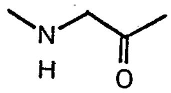

*1b*

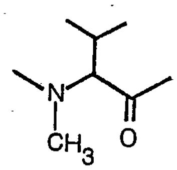

*1c*

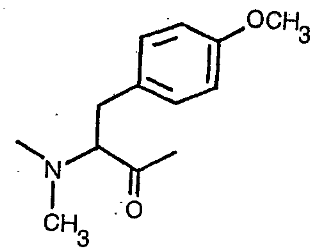

*1d*

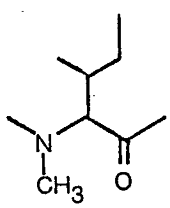

*1e*

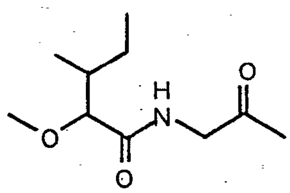

*1f*

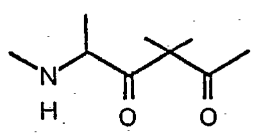

*1g*

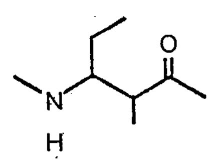

*1h*

h) il semble qu'il contient le groupement de sous-unité: glycyl-N-méthylisoleucylglycyl-N-méthylvalyl-N,O-diméthyltyrosyle; et
i) il possède la structure possible suivante:

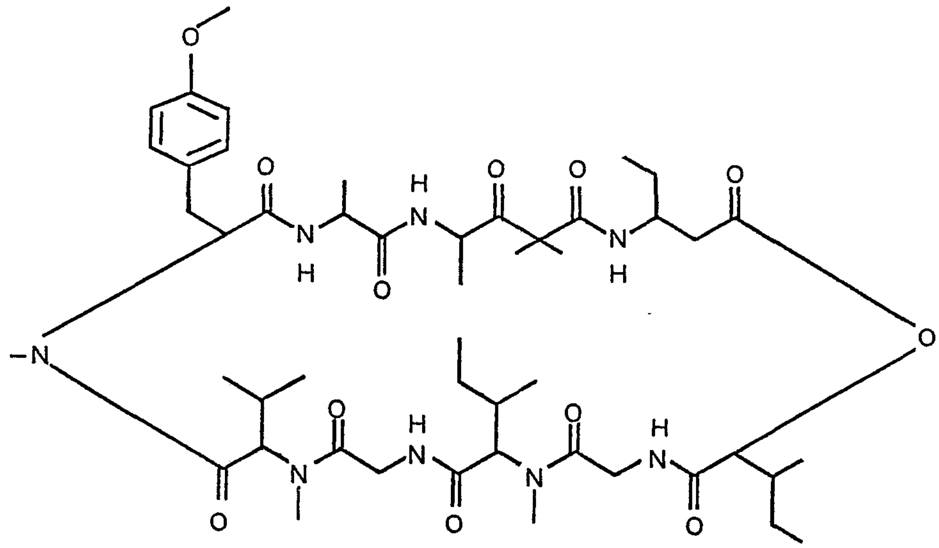

2

2. Majusculamide C suivant la revendication 1, en vue de l'utiliser comme fongicide.

3. Formulation fongicide comprenant, comme ingrédient actif, une quantité efficace de majusculamide C suivant la revendication 1 en association avec un ou plusieurs diluants ou supports acceptables du point de vue agronomique.

4. Formulation fongicide suivant la revendication 3, caractérisée en ce qu'elle est un concentrat acceptable du point de vue agronomique.

5. Procédé en vue d'inhiber un champignon pathogène des plantes, caractérisé en ce qu'il consiste à appliquer, au locus du champignon, une quantité efficace du composé suivant la revendication 1.

6. Procédé de préparation du composé suivant la revendication 1 à partir d'une association d'algues d'eau profonde contenant, comme constituant primaire, l'algue bleu-vert *Lyngbya majuscula*, caractérisé en ce qu'il consiste à extraire l'algue avec un solvant organique dans lequel le composé est soluble, puis isoler le composé par chromatographie.

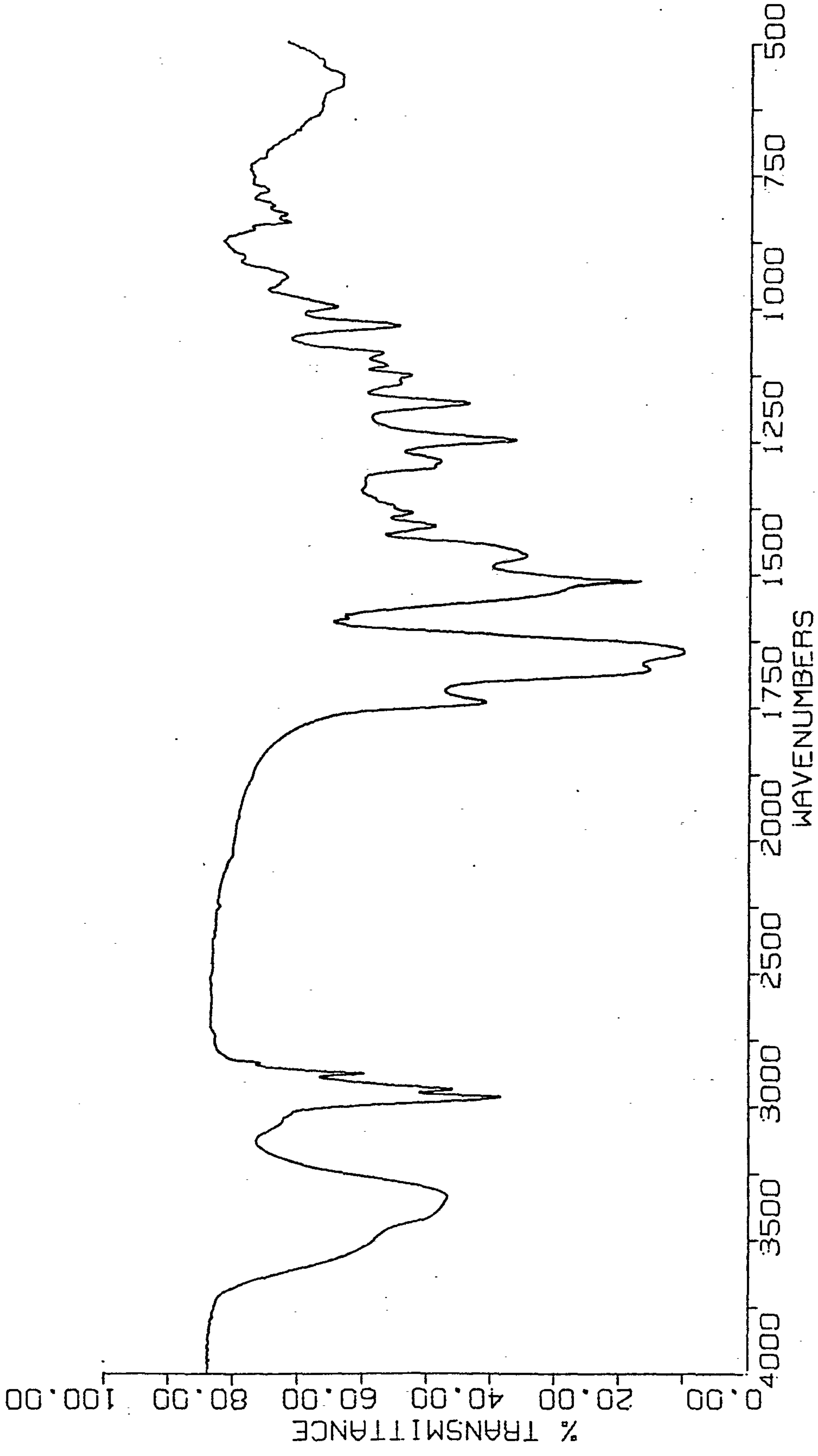

% TRANSMITTANCE
0.00
20.00
40.00
60.00
80.00
100.00
WAVENUMBERS
4000
3500
3000
2500
2000
1750
1500
1250
1000
750
500